# EUROPEAN PATENT APPLICATION

(11) **EP 2 009 533 A1**
(43) Date of publication of application: **31.12.2008**
(21) Application number: 08252200.4
(22) Date of filing: 26.06.2008
(51) Int. Cl.: G05B 23/02, A61M 5/00

(54) **Improvements to medical infusion pumps**

(30) Priority: 27.06.2007 US 769171
(71) Applicant: Animas Corporation, West Chester PA 19380 (US)
(72) Inventor: Hendrixson, Charles, West Chester, PA 19380 (US); Shipway, Ian Maxwell, Bryn Mawr, Pa 19010 (US); Quinlan, John, Spring City, PA 19475 (US); McLaughlin, Brian J., Media, PA 19063 (US); Getz, Steven, Malvern, PA 19355 (US)
(74) Representative: Tunstall, Christopher Stephen

(57) **Abstract**

Disclosed is a medical infusion device including a dedicated processing unit for detecting abnormal operation of the operation of the device. Specifically, a watchdog controller is employed as an independent monitor of the functioning of microprocessors used in the medical infusion device for controlling things such as RF communication, insulin infusion, and system integrity. Through the use of an independently-powered watchdog control system, the accuracy and reliability of the device is enhanced, resulting in greater assurance to the patient receiving periodic or continuous infusion of a drug.

## Description

### BACKGROUND OF THE INVENTION

### 1. Field of the Invention

The present invention relates, in general, to medical devices, and more particularly to methods and devices for measuring an analyte present in a biological system.

### 2. Description of the Related Art

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia which, if left uncorrected, can lead to ketosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

In order to more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid which is then analyzed by the meter. In most cases, the meter has a display screen which shows the BG reading for the patient. The patient may then dose themselves with the appropriate amount, or bolus, of insulin. For many diabetics, this results in having to receive multiple daily injections of insulin. In many cases, these injections are self-administered.

Due to the debilitating effects that abnormal BG levels can have on patients, i.e., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed.

Insulin pumps are generally devices which are worn on the patient's body, either above or below their clothing. These relatively small, unobtrusive devices typically store a quantity of insulin in a replaceable cartridge and include a processing unit, a display screen, and input functions such as buttons or a keypad. Such pumps may include the ability to run multiple insulin delivery programs, such as basal and bolus programs, to eliminate the need for injections of insulin via needles and syringes, by providing medication via an infusion device that can be worn by the patient for an extended period of time, usually in the range of 1-3 days.

Patients using insulin pumps typically have the ability to program insulin delivery times and amounts into their pump's software, and enter their BG values into the pump via a data input system to deliver boluses of insulin in response to their activities, such as exercise and meal intake. Alternatively, the BG meter and pump may be in communication to permit the meter to transmit the BG reading to the pump along with a recommended bolus value, or to permit the pump or user to determine the appropriate bolus of insulin, if any. While the convenience of an insulin pump may improve the lifestyle of the patient and lessen the imposition of their disease on their normal activity, such persons are still susceptible to experiencing symptoms of diabetes which may render them unable to operate their meter, pump, or both, thereby leaving them unable to self-administer the necessary bolus of insulin in response to abnormal BG levels.

A need exists, therefore, for a system of BG monitoring and insulin delivery that may provide additional assistance to diabetics experiencing highly abnormal BG levels and require a device that provide distinctive alarms during certain situations, in order to alert the user to the type of action that must be taken.

Medical pumps such as insulin infusion pumps are commercially available and may include the capability to deliver a carbohydrate insulin bolus in conjunction with a blood glucose correction bolus by simply adding the blood glucose portion to the delivery total. Users wanting to add the blood glucose correction bolus to the normal portion first have to calculate, then specify the percentage of the total bolus that approximates the blood glucose correction portion along with any additional desired normal bolus amount. Such a procedure requires the user to undertake an additional task, may be time consuming and has the potential to introduce errors.

System processors equipped with software "watchdog" routines that periodically check on the proper operation of other system processors are known in the art. Operation is typically by digital "handshaking" communications between the processors. For example, if one processor identified that another processor was not functioning properly, the former would attempt to alert the user by activating the alarm transducer(s) to which it was connected by means of the watchdog component.

As system hardware and software complexity has grown, it has become increasingly difficult and time-consuming to verify that software "watchdog" checks are adequate in all failure modes. Furthermore, such software checks may complicate development and verification of system software. For example, a minor change or enhancement in the software of one processor (e.g. an additional UI feature) could force a time-consuming re-verification of the entire software watchdog system.

### SUMMARY OF THE INVENTION

The present invention eliminates the need for the user to manually estimate the blood glucose correction amount of insulin by automatically adjusting the normal portion percentage of the combo bolus delivered. This reduces the amount of user intervention with their medical pump, providing additional reassurance that the system is managing their condition reliably.

A watchdog circuit may be used to ensure that the insulin pump provides the user with audio and vibratory alarms no matter what type of fault may occur between any of the other microcontrollers in the insulin pump device. The watchdog circuit therefore is intended to eliminate additional functions which otherwise would be put on each of the microcontrollers to check they are working correctly. The advantage of this present system and method is the elimination of undesirable, additional circuitry and complexity, while achieving greater reliability for insulin delivery. The present invention is therefore relates to a watchdog circuit that has may ensure that all microcontrollers in the insulin delivery device are functioning correctly. The watchdog circuit further may ensure that the highest volume audio alarm will be used to alert the user. Furthermore the watchdog circuit provides a method to periodically verify other clock signals in the insulin pump.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

Figure 1 is a simplified schematic view of an example embodiment of a system incorporating a measurement meter, a pump and a remote device such as a computer;

Figure 2 is a perspective view of the pump of Figure 1;

Figure 3 is a flow diagram showing different example delivery options available to a user of a medical pump;

Figure 4 is an example embodiment of a 'Bolus total' screen shot that may be provided to a user of the pump of Figure 2;

Figure 5 is an example embodiment of a 'Carb combo' screen shot that may be provided to a user of the pump of Figure 2;

Figure 6 is an example embodiment of a 'Combo total' screen shot that may be provided to a user of the pump of Figure 2;

Figure 7 is an example embodiment of a warning screen that may be provided to users of a pump such as the pump of Figure 2;

Figure 8 is a flow diagram showing a series of example screen shots that may be provided to the patient during use of a medical pump, such as the pump of Figure 2.

Figure 9 is a further flow diagram of example screen shots that may be provided to the user of a medical pump, such as the pump of Figure 2;

Figure 10 is an example screen shot of set-up information that may be displayed to a user according to an embodiment of the present invention;

Figure 11 is an example screen shot that may be displayed to a user to inform them that the requested dosage exceeds a two hour maximum limit, according to the present invention;

Figure 12 is a circuit block diagram detailing the internal components of the pump of Figure 1;

Figure 13 is a simplified block diagram of a watchdog circuit according to the present invention.

### DETAILED DESCRIPTION OF ILLUSTRATIVE EMBODIMENTS OF THE INVENTION

While preferred embodiments of the present invention have been shown and described herein, it will be obvious to those skilled in the art that such embodiments are provided by way of example only. Numerous variations, changes, and substitutions will now occur to those skilled in the art without departing from the invention.

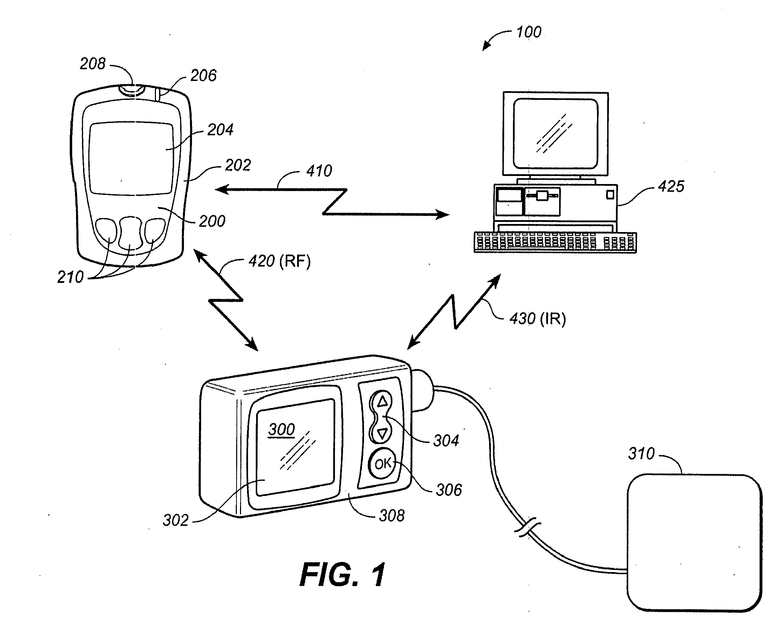

**Figure 1** shows a measurement system 100 comprising a meter/remote control 200, an external pump 300, an external device such as a PC 425. Meter/remote control 200 includes a housing 202, a display 204, an optional LED 206, a measurement interface 208 such as a blood glucose measurement interface and user operable buttons 210. Pump 300 includes a display 302, up/down arrow buttons 304, an 'OK' button 306, a housing 308 and communication to an infusion set 310. System 100 also may include bi-directional communication 410 between meter/remote control 200 and PC 425, bi-directional communication 420 between meter/remote control 200 and pump 300 and optional bi-directional communication 430 between pump 300 and PC 425.

Measurement system 100 illustratively comprises two main components, a meter/remote control 200 and an external pump device 300. System 100 may further include one or more external devices such as PC 425 for example. In one example embodiment, RF communication 420 may incorporate a unique communication protocol that has a learn mode or "pairing" mode which pairs meter/remote controller 200 and pump 300, in which the unique identification code of each communicating device is exchanged. Device "pairing" is a process in which a master i.e. meter/remote controller 200 learns who its slave is i.e. pump 300, and in which the slave learns which device its master is. Typically, only one meter/remote controller 200 and one pump 300 may be paired at a time.

In one exemplary embodiment, pump 300 is an insulin infusion pump delivering insulin through an infusion set 310 for subcutaneous infusion diabetes care. The menu-driven user interface of pump 300 is navigable and editable by use of the front panel up and down arrow buttons 304, and "OK" button 306. Output may be shown on display 302. The pump keys may be locked to prevent inadvertent pressing by holding both the up and down arrow buttons 304 until the "locked" message appears on display 302. Once locked, holding both the up and down arrow buttons 304 again unlocks the keys.

The small hand-held pump is often attached to the user's clothing by a holster. Pump 300 is optionally fully functional as a stand-alone pump as well as remotely operable by meter/remote control 200 using radio frequency (RF), or other forms of communication 420. Housing 202 may be plastic and ergonomically designed to be handheld. Housing 202 may also be constructed to be as RF "transparent" as possible. This is accomplished by the use of a custom design antenna assembled on a printed circuit board (PCB) that allows for the transmission and reception of RF by the meter/remote 200 while in the user's hand, to a pump 300 optionally located at a point on or near the user's body.

Measurement interface 208, for example a blood glucose-monitoring (BGM) portion of meter/remote control 200 may be an integrated meter and strip system, for the measurement of whole blood glucose with the strip (not shown) being a disposable device. Blood glucose data generated by meter/remote control 200 as well as other user inputs are used to calculate an insulin delivery. This information may be sent via RF communication 420 to pump 300, as well as being stored in the memory of meter/remote control 200. The combined data can be viewed on the meter/remote control display 204 or optionally downloaded to a PC 425. Meter/remote control 200 may optionally communicate to an external device such as a PC 425 or similar device in order to download and/or upload pump data. Communication 410 may be by means of a universal series bus (USB) for example, or by infrared (IR) communication.

A feature of meter/remote control 200 is the ability to communicate with and remotely control the insulin infusion pump by use of RF transmission and reception (bi-directional) 420. The meter/remote control 200 functions include the ability to display the current pump status. Since the meter/remote control 200 queries the pump 300 for its status, meter/remote control 200 can communicate pump errors, alarms, warnings and alerts on the display 204. For some alarms and warnings, meter/remote control 200 supports acknowledgment of the alarm or warnings thereby clearing pump 300 of the error. Meter/remote control 200 may optionally allow the user to generate commands to bolus from the pump, switch basal delivery programs, calculate and recommend bolus dosages.

Meter/remote control 200 is typically handheld, and functions as a convenient platform for the active management of an analyte of interest such as blood glucose for example, and optionally also functions as a remote control of an external pump 300, such as an insulin infusion pump for example. Although reference will be made to glucose monitoring and an insulin pump, it will be apparent to those skilled in the art that the present invention will be equally applicable to the measurement of any analyte and may be incorporated within any type of medical pump.

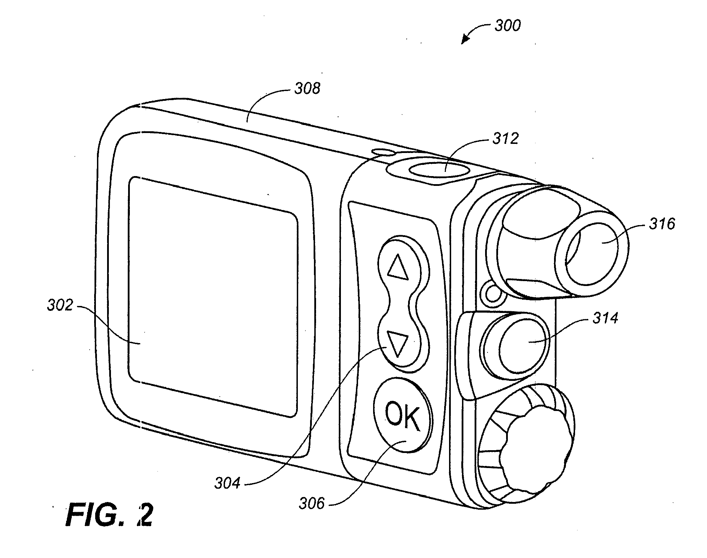

Turning to certain aspects of pump 300, **Figure 2** is a perspective, more detailed view of pump 300 of Figure 1, including a display screen 302, up/down arrow buttons 304, an 'ok' button 306, a housing 308, a contrast button 312, a bolus button 314 and a connector 316 to infusion set 310 (shown in Figure 1).

Pump 300 is intended to provide insulin infusion to the user according to a selected user or healthcare professional configurable basal program. Insulin infusion pump 300 can be used to manage diabetes by mimicking the way a healthy pancreas delivers insulin to the body. The way a patient's body uses insulin can be affected by many things such as lifestyle, exercise, weight loss or gain, therefore the basal rate that maintains blood sugar levels between meals may need to be modified, as a basal rate that is too high or too low may adversely affect blood glucose levels. In addition to a continuous, low-level basal rate, a higher level bolus dose can be programmed to be delivered to compensate for food eaten or to correct for high blood glucose levels. The bolus dosage uses factors such as manually entered carbohydrate, measured blood glucose values, insulin sensitivity factor (ISF) for the current time of day, insulin on board (IOB) at the current time or the amount of insulin delivered but not yet absorbed by the body, and insulin to carbohydrate (I:C) ratios at the current time. A Healthcare Professional (HCP) or diabetes specialist would typically determine such factors; in particular ISF may be variable at different times of day for example, and IOB varies from person to person and can vary due to infusion site as well as activity levels. Consideration of IOB during calculation of bolus dosages may help minimize the risk of hypoglycemia.

Pump 300 may also provide personal reminders such as when to make a blood glucose measurement, bolus reminders and alarm notifications to the user. Optionally, pump 300 may send history records including alarms, settings, insulin delivery and pump operation information to meter/remote control 200 via radio frequency (RF) communication 420.

Some commercially available pumps currently have the capability to deliver a carbohydrate insulin bolus in conjunction with a blood glucose correction bolus (BG bolus) simply by adding the blood glucose correction portion to the delivery total. However, this method has the effect of splitting the blood glucose increase between the 'normal' and 'extended' portions of the insulin delivery, requiring the patient to first calculate, then specify the percentage of the total bolus that approximates the BG correction portion along with any additional desired normal bolus amount. Once this has been programmed into the pump, the user may have the option of whether to deliver the bolus immediately as a normal bolus or as a combined or 'combo bolus'. The combo bolus is a feature enabling the normal bolus to be delivered immediately, and the remainder is delivered over an extended period of time i.e. up to 12 hours later that can be beneficial for high fat meals, 'grazing' or gastroparesis for example.

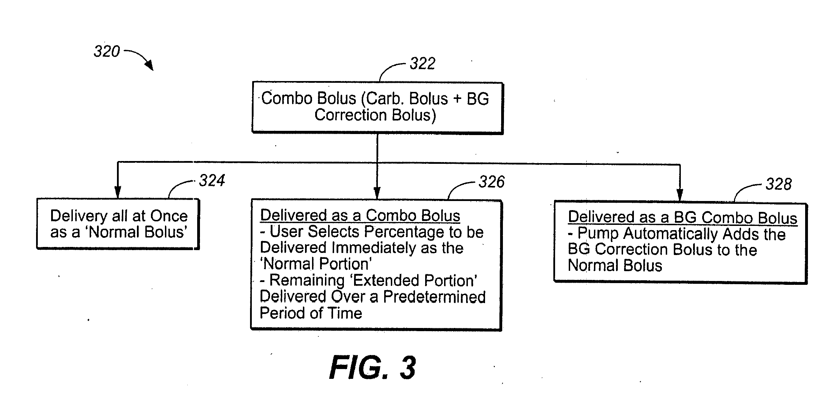

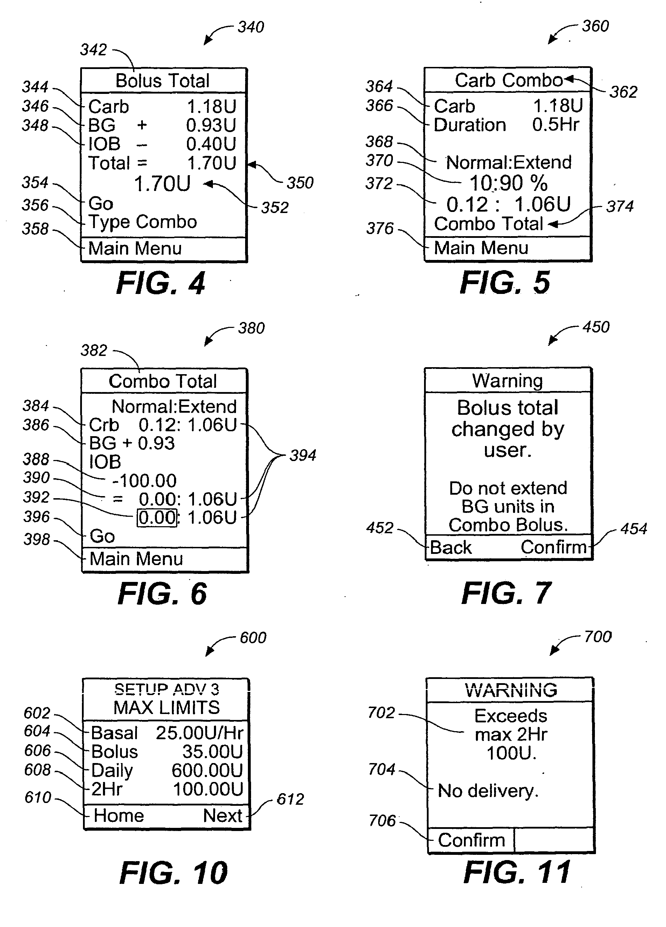

A more desired effect is to have the entire blood glucose (BG) correction bolus (BG bolus) immediately, during the normal portion of the combo bolus. Therefore an advantage of the present invention is to allow the blood glucose correction portion of the combo bolus to be delivered immediately and the remainder of the combo bolus to be delivered in a way determined by the user. If the user would like a percentage of the carbohydrate bolus to also be delivered immediately, then this percentage may be programmed to be delivered in addition to the blood glucose correction bolus. This ensures that the patient receives the blood glucose portion of the bolus immediately, yet still allows the convenience of a single programming sequence for both types of bolus. In the example embodiment provided, the blood glucose correction bolus becomes part of the normal portion of the combo bolus. Figure 3 provides a flow diagram outlining the different insulin delivery options, and Figures 4 to 9 provide example screen shots of information that may be displayed to a patient to enable navigation through the 'BG combo' feature of the present invention.

**Figure 3** is a flow diagram 320 showing different example delivery options available to a user of a medical pump such as an insulin delivery pump for example, including a combo bolus 322, a first option 324 where delivery is all together as one normal bolus, a second delivery option 326 which provides for delivery of a combo bolus, and a third delivery option 328 providing delivery as a BG bolus according to the present invention.

First and second delivery options, 324 and 326 respectively are features already provided by some commercially available pumps. Third delivery option 328 is provided as an example embodiment of the present invention whereby the software of pump 300 calculates the BG bolus from recent blood glucose measurement data, and this BG bolus may automatically be delivered in addition to the normal portion of the combo bolus. Provision of an automated system relieves the patient from having to estimate the percentage of the combined delivery that closely approximates the blood glucose correction portion. Provision of a BG combo bolus therefore eliminates at least one user step, thereby simplifying the measurement and dosing procedure. Most patients would rather their medical devices provided them with reliable control of their condition with minimal intervention. Reducing the number of user steps also reduces the likelihood of errors occurring due to a patient's own calculation.

**Figure 4** is an example embodiment of a screen shot 340 that may be provided to enable the user to better manage the blood glucose increased amount, showing a screen title 342 i.e. 'Bolus total' in this example, a carb (carbohydrate) bolus 344, a BG (blood glucose) bolus 346, an 'insulin on board' level 348, a total value 350, a user-entered value 352, a 'Go' feature 354, a 'Type combo' indicator 356 and an option to return to a main menu 358.

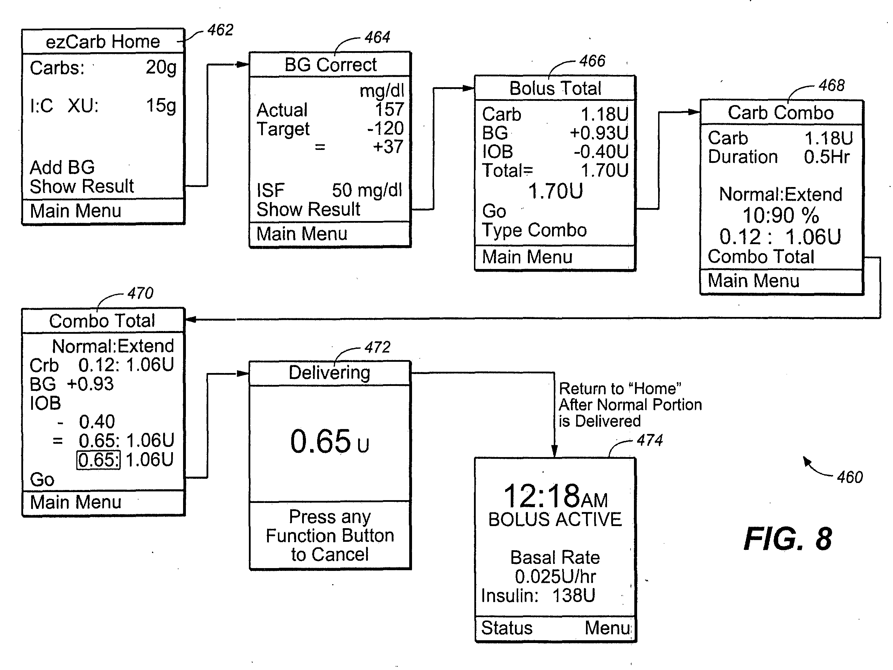

In the example embodiment of a BG combo bolus feature according to the present invention, it is intended that the patient would use the automatically calculated total value 350. However the user is free to enter any value they wish into 'user-entered value' 352. Once a value is entered, the user then selects 'Go' 354 to begin immediate delivery of the combo bolus with the BG bolus portion being delivered with the normal portion. A full series of example screen shots is provided in figure 8.

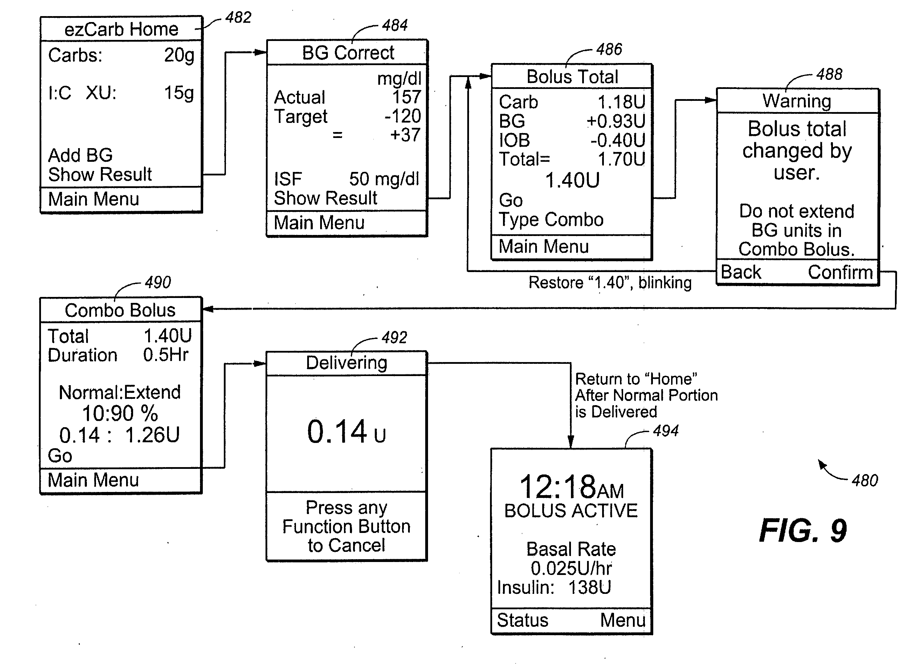

If the user enters an amount corresponding to the automatically calculated total value 350, then the BG combo operation proceeds as will be described in relation to Figure 8. If however, the user enters a different value from total value 350 provided into user-entered value 352, then the BG combo operation will revert to a typical combo operation which does not consider BG compensation, such as delivery option 326 shown in Figure 3, which is also discussed further in relation to Figure 9. This is due to the fact that the intentions of the user cannot be assumed, therefore a normal combo bolus is delivered.

**Figure 5** shows a further example screen shot 360 of information that may be displayed to a user, including a title 362 i.e. 'Carb Combo', a dosage for the carb bolus 364, a duration period 366 e.g. 0.5 hours as shown, a ratio between normal and extended delivery 368, a percentage ratio 370, an actual dosage ratio 372, a 'combo total' option 374 and a return to main menu 376.

'Carb combo' screen 362 allows the user to set the proportion of the total bolus to be delivered either immediately as a normal bolus, or extended over a predefined period of time as the extended bolus. Each of the parameters displayed are changeable using the up/down arrow keys 304 and 'ok' button 306. For example, duration period 366 may be increased or decreased in half hour intervals; percentage ratio 370 may be modified in 5% increments. Selecting 'combo total' 374 moves the display forward to the next screen, as shown in Figure 8. Optionally the user may return to the main menu at any point in the procedure by selecting 'main menu' option 376.

**Figure 6** shows a further example screen shot 380, including a title 382 i.e. 'Combo total', a partition of the carb bolus between normal and extended portions 384, a BG correction bolus 386, an insulin on board (IOB) value 388 which may be taken from the 'Bolus total' screen of Figure 4, a calculated delivery volume 390, an actual delivery volume 392, a carb extended portion 394, a 'Go' selection 396 and a return option to the main menu 398.

'Combo total' screen 382 shows the division between the normal portion and the extended portion for each of the carb bolus 384 and the blood glucose correction bolus 386, and shows the amount of BG bolus taken from 'Bolus total' screen described in relation to Figure 4. Each of the carb bolus 384, BG bolus 386, IOB value 388 and the carb extended portion 394 are shown on 'Combo total' screen 382 optionally to show their status only, and may not be editable at this stage. Calculated delivery volume 390 is the calculated amount for the normal portion of the combo bolus delivery, and is the total of the Carb, BG and IOB values. This calculated delivery volume 390 represents the expected value to be entered by the user as the normal portion of the combo bolus. Actual delivery volume 392 allows the user to define the normal portion of the combo bolus. In the embodiment provided it is anticipated that the user will enter the same amount as the calculated delivery volume 390, however the user may enter any value as long as it is within a valid range. Highlighting and selecting 'Go' option 396 may begin immediate delivery of the combo bolus, with the BG correction bolus being delivered with the normal portion.

If the user enters a bolus total that is different from the recommended amount, then a warning screen 450 such as the example provided in **Figure 7** may be displayed to the user in order to confirm 454 or optionally abort the change by selecting a back option 452. Warning message 450 may optionally be displayed from 'Bolus total' screen 342. Selecting back option 452 will return the user to 'Bolus total' screen 342 where the last entered bolus value will be restored and blinking, providing the user with the opportunity to change the bolus value. Selecting confirm 454 acknowledges that the user has chosen to ignore the BG content and will therefore proceed with a typical, non-BG combo. This procedure is discussed further in relation to Figure 9.

**Figure 8** is a flow diagram 460 of example screen shots that may be provided to the user of a medical pump such as pump 300 used for insulin infusion. An initial 'exCarb Home' screen 462 may show information such as a carbohydrate value, the patient's insulin to carbohydrate ratio, and provide routes to additional options such as 'Add BG' and 'Show result'. BG Correct screen 464 shows the actual and target blood glucose values along with the insulin sensitivity factor (ISF). A patient's HCP may recommend the use of different target ranges for different times of day. If, for example, a blood glucose measurement provides a result that is within the predetermined range for the time of day, then the pump will not need to calculate a BG correction bolus.

Selecting 'Show result' takes the user to the 'Bolus total' screen 466 (as described in relation to Figure 4) from which selecting 'Go' moves the user to 'Carb combo' screen 468 (as described in relation to Figure 5) where the user can modify the proportion of normal to extended delivery of bolus as well as the duration of the extended portion of the combo bolus. Selecting 'Combo total' whilst within the 'Carb combo' screen 468 takes the user to the 'Combo total' screen 470, where selecting 'Go' initiates the immediate delivery (screen 472) of the normal portion of the combo bolus. Optionally, pressing any button on the front of pump 300 during a bolus delivery will stop the delivery and a corresponding warning screen (not shown) may be displayed to the user, providing them with the option to confirm termination of the bolus delivery.

In the exemplary embodiment provided, the normal portion of the combo bolus is equal to 0.65 units of insulin, and display of Delivery screen 472 assures the patient that delivery is taking place. The pump may optionally 'beep' to confirm the start of delivery, and optionally also when delivery is complete. This feature may be enabled or disabled at any time by the user. Following delivery of the normal portion of the combo bolus, the pump display 302 (and optionally duplicated on meter/remote control display 204 for additional user convenience) may return to the 'Home' screen 474. Home screen 474 may display information such as the current time, the status of the pump i.e. whether or not there is an active bolus and the basal flow rate setting for example.

**Figure 9** is a further flow diagram of exemplary screen shots that may be provided to the user of a medical pump such as pump 300 of the present example, if the user enters a value in 'Bolus total' screen 340 that is different from the value automatically calculated by the pump software. Screens 482, 484 and 486 are identical to 462, 464 and 466 of Figure 8, however, selecting 'Go' in the example embodiment provided in Figure 9 triggers warning screen 488 due to the incompatibility of values entered in the Total Bolus screen. From warning message 488 the user is provided with two options: to either accept or reject this change that has been identified. As described previously in relation to Figure 7, rejecting the value takes the user back to the previous screen in order to re-enter the value. Accepting the entered value causes the bolus to revert back to a normal 'Combo bolus', screen 490, rather than a 'BG-Combo bolus' of the present invention.

**Figure 10** is an illustrative screen shot 600 of set-up information that may be displayed to a user in order to configure a specific maximum delivery limit, including a basal flow rate 602, a bolus amount 604, a daily maximum total 606, a two hour maximum limit 608, a 'Home' option 610 and a 'Next' option 612.

**Figure 11** is an illustrative screen shot 700 that may be displayed to a user to inform them that a requested dosage exceeds a predefined maximum limit, according to the present invention, including a warning message 702, a statement 704 and a 'Confirm' option 706.

Referring now to both Figures 10 and 11, medical infusion pumps, such as pump 300 described herein may typically incorporate a 'daily' maximum total infusion 606 that covers a 24 hour period, as well as optional maximum limitations on programming individual bolus amounts 604 and basal flow rates 602. Setting a maximum limit for total daily insulin delivery 606 enables patients to maintain control over their daily amounts as well as preventing any accidental over-dosing. Incorporation of a 24-hour maximum limit 606 therefore aims to mitigate issues relating to patient forgetfulness or confusion. However, medical systems such as system 100 of Figure 1 that comprise a pump 300 and a meter/remote control 200 that can be used to operate pump 300 remotely, introduces the potential for an unauthorized user to gain access to meter/remote control 200 and attempt to deliver a bolus amount either accidentally or maliciously, without the knowledge of the pump wearer. Such interference could magnify the problem of over dosing, particularly if the patient has relatively low blood sugar levels at the time, as even a small bolus delivered may have a significant effect and potentially cause problems. Therefore, incorporation of a predefined short-term maximum limit, such as a two-hour maximum limit 608 of the present invention aims to alleviate this problem.

Incorporation of a two-hour maximum limit 608 not only provides the pump patient with a finer time resolution in which to control the previously identified risks, such as over dosing as a result of forgetfulness or confusion, but also aims to virtually eliminate any potential harm caused by an 'unauthorized user' which may stem from the remote capabilities of such a monitoring system. A short timescale maximum delivery limit such as two-hour maximum limit 608 allows the user to set a control or threshold value for maximum delivery in accordance with their prescribed insulin regimen, thus preventing over dosing. The short-term maximum delivery limit 608 of the present invention is based on short time periods i.e. less than 24 hours, and causes the pump to prevent any deliveries that exceed this maximum limit within the predefined time constraints. Operation of the maximum delivery limit 608 may be communicated to the user via both the pump display 302 as well as the meter/remote control display 204.

If a user inadvertently duplicates a bolus that causes the accumulated insulin delivery amount to exceed the short-term maximum limit 608, the pump 300 will disallow the bolus and display a warning message to the user, such as warning message 700 for example. Warning message 700 includes both the reason for disallowing the bolus 702 i.e. stating that it would exceed the maximum limit 608, as well as the action that will be taken 704 i.e. 'No Delivery'. Warning screen 700 may optionally be displayed on both the pump display 302 and the meter/remote control display 204 for added convenience. If a user receives such a message, they will be able to review the pump history to verify that the insulin dose had already been delivered.

Provision of a short-term maximum limit 608 of the present invention therefore effectively protects the user from over-dosing. Furthermore, a patient is also protected against any accidental or malicious bolus delivery by an unauthorized user gaining access to meter/remote control 200.

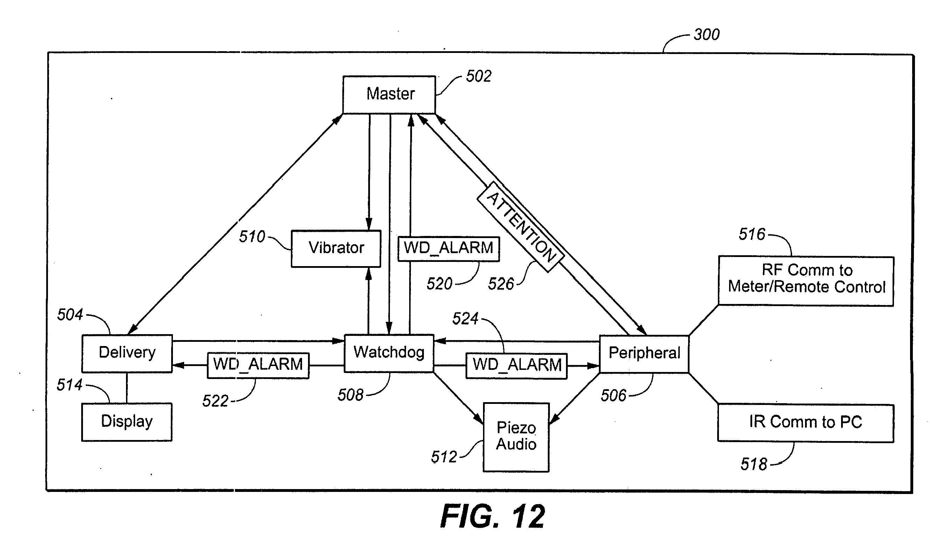

**Figure 12** shows a circuit block diagram detailing the internal components of pump 300 including a master microcontroller 502, a delivery microcontroller 504, a peripheral microcontroller 506, a watchdog microcontroller 508, a vibrator 510, a piezo audio 512, a display 514, an RF communication 516, an IR communication 518 and WD_ALARM signals 520, 522, 524 and an attention signal 526.

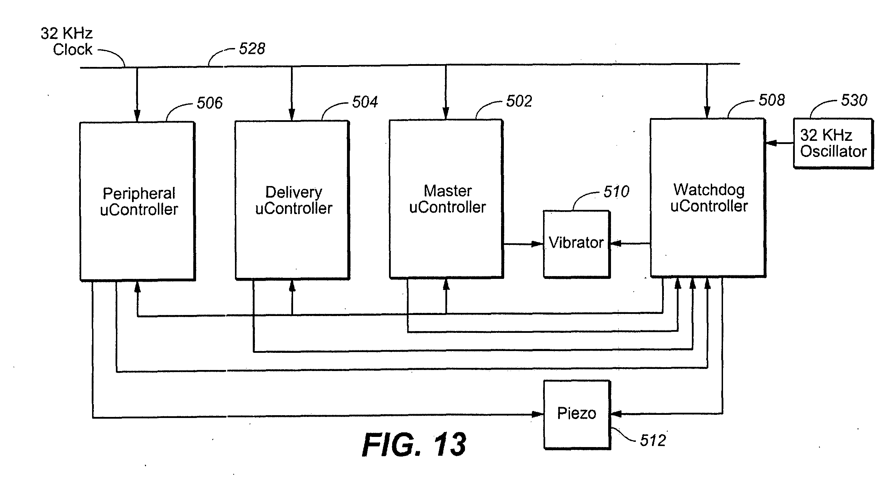

**Figure 13** shows a simplified block diagram of the watchdog circuit according to the present invention, including a watchdog microcontroller 508, a Master microcontroller 502, a Delivery microcontroller 504, a Peripheral microcontroller 506, a vibrator 510, a piezo audio signal 512, a 32KHz Clock line 528 and a 32KHz oscillator 530.

Referring now to Figures 12 and 13, the illustrative embodiment of a pump 300 provided has 3 microcontrollers (alternatively known as processors), the Master 502, the Delivery 504 and the Peripheral 506, and each of these microcontrollers sends a signal to the watchdog Monitor microcontroller 508 at least once per hour to indicate that they are functioning correctly. Such a signal may consist of a high to low back to high transition three times within a 10ms period, for example. This pattern may be sent to the watchdog monitor more frequently than once per hour if necessary.

The watchdog microcontroller monitors each of the three digital inputs from the Master 502, Delivery 504 and the Peripheral Microcontrollers 506 and will drive the piezo audio alert 512 and/or vibrator 510 to the highest (loudest) level and most frequent vibration period under 'alarm conditions' if any of the three inputs does not receive this predefined pattern e.g. three negative pulses within the specified time period e.g. one hour.

Under 'normal conditions', typical types of 'usual' or 'expected' errors and alarms include, but are not limited to occlusion and identification of an empty cartridge for example. Warnings such as low cartridge, low battery and delivery halted or suspended (must be confirmed) may also be signaled using vibrator 510 and/or piezo audio 512 alerts. Pump 300 alarms and errors may be signaled using vibrator 510 and/or a progressive audio signal 512 that gets progressively louder. Optionally both vibrator 510 and piezo audio signal 512 may be used. Other pump alarms such as the need to replace the battery, auto-off or call service may be signaled using the piezo audio alert 512. Under normal pump operation, the user may optionally control characteristics of vibration 510 and audio 512 transducers to enhance their personal discretion and comfort by adjusting programmed settings in the pump, for example setting audio transducer 512 to operate at a reduced volume, alternatively audio 511 and/or vibration 510 transducers may be selectively disabled, the activation time and sequence of both transducers may be selected to be unobtrusive or the tonal characteristics of the audio alarm may be selected to be unobtrusive.

Under normal pump operation, vibration 510 and audio 512 transducers are controlled by Master microcontroller 502, whereas under alarm conditions watchdog microcontroller 508 controls vibration motor 510 and audio transducer 512 through a parallel control path. This control path allows the watchdog microcontroller 508 to override the control of other devices under alarm conditions. An error of any type detected by watchdog microcontroller 508 may trigger operation of vibrator 510 and/or piezo audio 512 to alert the user to the fault. A message may also be displayed to the user on display 302 providing information regarding the type of fault or error that has occurred. In the example embodiment provided, when a fault is identified with one of the Master 502, Delivery 504 or Peripheral 506 microcontrollers, watchdog microcontroller 508 will echo the pattern received i.e. the three negative pulses in the example provided, back to the Master microcontroller on a separate "WD_ALARM" signal line 520 immediately after it has received the pulses from the Master microcontroller 502. This echo verifies that the watchdog microcontroller 508 is operating properly. watchdog microcontroller 508 will also provide a series of pulses on the "WD_ALARM" signal line 520 to indicate which of the microcontrollers has failed. "WD_ALARM" signal lines 520, 522 and 524 are connected to the Master 502, Delivery 504 and Peripheral 506 microcontrollers respectively.

Under alarm conditions, watchdog microcontroller 508 has the ability to override the normal mode settings and control the transducers in such a way as to provide the best assurance that the user will recognize the alarm. Watchdog microcontroller 508 therefore includes a reset signal and power monitoring circuit that is independent of that used by the Master 502, Delivery 504 and Peripheral 506 microcontrollers. This independent circuit allows watchdog microcontroller 508 to alarm if there is a malfunction of the primary reset circuitry.

Incorporation of a watchdog microcontroller circuit 508 of the present invention provides the advantage of having an independent microcontroller tasked with the sole function of monitoring and checking the status and performance of the other microcontrollers within the system i.e. Master 502, Delivery 504 and Peripheral 506 microcontrollers of the present example. Provision of such an independent microcontroller purely for a supervision function means that the other microcontrollers within the system are in no way compromised with the burden of undertaking this additional task alongside the normal duties expected from them. Furthermore, provision of an independent watchdog microcontroller circuit 508 enables minor changes in the development of a product, such as enhancement in the software of one processor for example, without having to go through a time consuming full re-verification of the entire software watchdog system.

The watchdog microcontroller 508 will generally include a 32.768KHz crystal oscillator 530 as shown in Figure 13 to ensure continued operation of watchdog microcontroller 508 even if something interrupts 32KHz clock line 528. The watchdog microcontroller can perform periodic checks on the 32KHz clock 528. If this check fails, watchdog microcontroller 508 should generate an alarm. If Delivery Microcontroller 504 is operational, a signal will be sent to Delivery Microcontroller 504 on "WD_ALARM" signal line 522 so that a "Call for Service" screen may be displayed on display 514.

The watchdog microcontroller 508 may optionally produce a short beep and/or a short vibration during power-up (i.e. when the battery is changed). This short beep and vibration is verification that the watchdog microcontroller 508, the piezo driver circuit 512 and the vibration motor driver circuit 510 are operational. This beep and vibration should be coordinated to occur after the other power on beeps and vibration from the Master microcontroller 502. Periodic short beeps and short vibrations may also be generated during a priming operation to verify the operation of the piezo 512 and vibration motor 510 on a once per use basis.

In alternate embodiments, the watchdog circuit 508 may have its own independent power supply such as a battery so that it may alarm even when there is a main power supply failure. Optionally the watchdog circuit 508 may have an independent alarm transducer(s). Optionally, the watchdog circuit 508 may independently disable the delivery motor circuit 504.

In order to conserve battery power, pump 300 will typically implement a sleep/idle mode when not in use, slowing clock signals and disabling processor modules. During normal operation, pump 300 limits RF communication, inactivates display 302 after a specific timeout period and turns off delivery hardware when not in use.

As discussed earlier, the present invention is not restricted to use with insulin infusion pumps, and may be used in medical pumps generally. Therefore in more general terms, the pump system may have "N" microcontrollers, where each of the N microcontrollers signal the watchdog monitor microcontrollers within specified periods of time to indicate that they are functioning correctly. The watchdog microcontrollers will be able to address N unique microcontrollers with a message indicating which microcontrollers has failed to signal it within a specified period of time, so that each of these N microcontrollers could control any indication devices under their control, and provide alert information to the end user as to which of the N devices has failed. Each of the N microcontrollers could signal the central watchdog microcontrollers, via a low active pulse {either state or edge triggered} every "T" minutes. The watchdog microcontrollers monitors each of the N unique lines. If the watchdog microcontrollers does not recognize a unique low active pulse and/or detects that the line is driven permanently low, it may trigger an Alarm Condition.

A watchdog microcontroller circuit that is independent of all other processors in an Insulin Pump and has control of the piezo or speaker audio alarm and a vibrator motor is provided. The watchdog circuit receives a periodic digital signal from each of the Microcontrollers in the Insulin Pump. If one or more of the other Microcontrollers do not provide the watchdog circuit with the pre-defined periodic signal, the watchdog circuit will provide an alarm to the user to identify that the Pump is not working properly. The watchdog circuit will also contain its own timebase so that the circuit may perform periodic accuracy checks on other timebase signals in the Insulin Pump.

It will be recognized that equivalent structures may be substituted for the structures illustrated and described herein and that the described embodiment of the invention is not the only structure which may be employed to implement the claimed invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. An drug infusion device, comprising:
a housing,
a drive control circuit disposed within the housing,
a drive motor in electronic communication with the drive control circuit,
one or more processing units,
a screen display for receiving input and in electronic communication with at least one of the one or more processing units, and
a watchdog processor for monitoring each of the one or more processing units.

2. The infusion device of claim 1 comprising an alarm control circuit.

3. The infusion device of claim 2, wherein the watchdog processor is configured to transmit an alarm signal to the alarm control circuit.

4. The infusion device of claim 1, wherein the alarm control circuit comprises one or more of an audible alarm signal, a vibratory alarm signal, and a visual alarm signal.

5. The infusion device of claim 1, wherein each of the one or more processing units is configured to send a signal to the watchdog processor on a predefined periodic basis.

6. The infusion device of claim 5, wherein the watchdog processor is configured to transmit an alarm signal to the alarm control circuit if a signal is not received from each of the one or more processing units on the predefined periodic basis.

7. The infusion device of claim 1, wherein each of the one or more processing units is configured to send a signal to the watchdog processor indicating a specific error condition.

8. The infusion device of claim 7, wherein the watchdog processor is configured to transmit an alarm signal to the alarm control circuit if a signal indicating a specific error condition is received from any of the one or more processing units.

9. The infusion device of claim 1, wherein the watchdog processor is powered by a watchdog power supply that is independent from a power supply that powers any of the one or more processing units.

10. The infusion device of claim 9, wherein the watchdog power supply provides power to the alarm control circuit.

11. The infusion device of claim 1 comprising a remote controller, the remote controller comprising one or more remote control processing units therein.

12. The infusion device of claim 11 comprising at least one communication control processor disposed within the housing for processing communication between the infusion device and the remote controller.

13. The infusion device of claim 11 wherein the communication between the infusion device and the remote controller employs a radio frequency (RF) protocol.

14. The infusion device of claim 11 wherein the remote controller comprises a blood glucose meter having a remote display.

15. The infusion device of claim 11 wherein the remote controller is configured to generate an audible alarm, a vibratory alarm, or a visual alarm on the remote display in response to receiving an instruction from the alarm control circuit.

16. The infusion device of claim 11, wherein the watchdog processor is configured to transmit an alarm signal to the alarm control circuit if a signal is not received from any of the remote controller processing units on the predefined periodic basis.

17. The infusion device of claim 11, wherein each of the one or more remote controller processing units is configured to send a signal to the watchdog processor indicating a specific error condition.

18. The infusion device of claim 17, wherein the watchdog processor is configured to transmit an alarm signal to the alarm control circuit if a signal indicating a specific error condition is received from any of the one or more remote controller processing units.

19. The infusion device of any of claims 8 and 17, wherein the alarm control circuit is configured to determine an alarm type based on the alarm signal.

20. The infusion device of claim 19, wherein the alarm type comprises an audible alarm, a vibratory alarm, a visual alarm, and combinations thereof.

21. The infusion device of claim 20, wherein the audible alarm comprises audible tones of varying volume.

22. The infusion device of claim 20, wherein the audible alarm comprises a low-volume audible tone, or a high-volume audible tone.
